# EUROPEAN PATENT SPECIFICATION

(11) **EP 3 973 966 B1**
(45) Date of publication and mention of the grant of the patent: **26.02.2025**
(21) Application number: 20198157.8
(22) Date of filing: 24.09.2020
(51) Int. Cl.: A61K 33/30, A61K 9/00, A61K 9/12, A61K 33/42, A61K 36/23, A61K 36/235, A61K 47/10, A61K 47/20, A61K 47/32, A61K 47/36, A61K 47/38, A61P 31/14, A61K 31/045, A61K 31/05, A61K 31/35, A61K 38/40, A61K 6/00

(54) **HYDROXYAPATITE COMPOSITION FOR TREATMENT AND/OR PREVENTION OF A VIRUS INFECTION**
HYDROXYAPATITZUSAMMENSETZUNG ZUR BEHANDLUNG UND/ODER VORBEUGUNG EINER VIRUSINFEKTION
COMPOSITION D'HYDROXYAPATITE POUR LE TRAITEMENT OU LA PRÉVENTION D'UNE INFECTION PAR UN VIRUS

(43) Date of publication of application: 30.03.2022
(73) Proprietor: Dr. Kurt Wolff GmbH & Co. KG, 33611 Bielefeld (DE)
(72) Inventor: SCHULZE zur WIESCHE, Erik., 33611 Bielefeld (DE); STARK, Miriam., 33611 Bielefeld (DE); DOERRENBERG, Eduard Richard., 33611 Bielefeld (DE)
(74) Representative: Ter Meer Steinmeister & Partner

(56) References cited:
- EP-A1- 0 344 832
- EP-A1- 3 888 629
- WO-A1-2018/145966
- WO-A2-2005/074947
- JP-A- 2007 302 587
- US-A1- 2004 033 260
- US-A1- 2004 086 575
- YU-A- 40 103
- MEISTER TONI LUISE ET AL: "Mouthrinses against SARS-CoV-2 - High antiviral effectivity by membrane disruption in vitro translates to mild effects in a randomized placebo-controlled clinical trial", VIRUS RESEARCH, AMSTERDAM, NL, vol. 316, 2 May 2022 (2022-05-02), XP087064394, ISSN: 0168-1702, [retrieved on 20220502], DOI: 10.1016/J.VIRUSRES.2022.198791
- "Supplementary Methods" as mentioned on page 3 under item 2.2. "Randomized placebo-controlled clinical trial" of Meister et al., Virus Research, 316
- DATABASE GNPD [online] MINTEL; 29 June 2016 (2016-06-29), ANONYMOUS: "Mouthwash", XP055676610, retrieved from https://www.gnpd.com/sinatra/recordpage/4090209/ Database accession no. 4090209
- MONMATURAPOJ NARUPORN ET AL: "Antiviral activity of multifunctional composite based on TiO2-modified hydroxyapatite", MATERIALS SCIENCE AND ENGINEERING C, ELSEVIER SCIENCE S.A, CH, vol. 92, 19 June 2018 (2018-06-19), pages 96 - 102, XP085462477, ISSN: 0928-4931, DOI: 10.1016/J.MSEC.2018.06.045

## Description

### Field of the invention

The present invention is directed to a composition comprising 0.001 to 40 wt.-% of hydroxyapatite for use in the treatment and/or prevention of a virus infection.

### Background of the invention

Viruses belonging to the family of *Coronaviridae* were first characterized in the 1960's. Virus infections belonging to this family are common in all mammal species including humans. The SARS-CoV-2 outbreak made it evident that novel pharmaceutical compositions are needed to treat and prevent the widespread of virus infections in general, and in particular infections with the SARS-CoV-2.

Corona viruses are RNA viruses with a linear ss-RNA (single-stranded RNA) that are transmitted via droplet infection and result i.a. in fever and respiratory problems. There are several species that are relatively harmless and result in only acute and mild symptoms or take an asymptomatic course (HCoV-OC43, 229E, HKU1, and HL63). On the other hand, SARS-CoV (Severe Acute Respiratory Syndrome Corona Virus), MERS-CoV (Middle East Respiratory Syndrome Corona Virus) and SARS-CoV-2 are Corona viruses that can lead to severe respiratory and lung infections. So far there are only very limited treatment or prophylaxis options, except for exposure prophylaxis. Some mouth rinse compositions have been investigated *in vitro* by Steinmann et al., The Journal of Infectious Diseases 2020, XX, pp 1-4 for the activity against SARS-CoV-2. JP 2007 302587 A discloses a silver-hydroxyapatite suspension useful for preventing avian influenza virus infection.

MONMATURAPOJ NARUPORN ET AL: "Antiviral activity of multifunctional composite based on TiO2-modified hydroxyapatite", MATERIALS SCIENCE AND ENGINEERING C, ELSEVIER SCIENCE S.A, CH, vol. 92, 19 June 2018 (2018-06-19), pages 96-102 discloses that hydroxyapatite-titania composite (HA/TiO2) inhibits H1N1 infection in cells.

YU 40 103 A discloses that hydroxyapatite has a high affinity for binding surface proteins S1 and S2 of the SARS virus and is therefore suitable for its removal from skin and surfaces.

In view of the above, it was an object of the present invention to provide a novel, safe, and effective composition for the treatment and/or prevention of a virus infection in general and in particular for the treatment and/or prevention of SARS-CoV-2.

### Summary of the invention

These objects have surprisingly been solved by a composition comprising 0.001 to 40 wt.-% of hydroxyapatite.

It has surprisingly been found that according to the above aspect, infections with a virus can be prevented or the probability of infection can at least significantly be reduced. In addition, and in accordance with the above aspect, the virus count in the respiratory tract is significantly reduced so that an infected person is no longer infectious towards third persons. The invention is defined in the claims.

The invention relates to a composition comprising 0.001 to 40 wt.-% of hydroxyapatite for use in the treatment and/or prevention of an enveloped virus infection, further comprising 0.001 to 5 wt.-% of a surfactant, wherein the composition is administered as a mouth rinse or as an aerosol by spraying, wherein the composition does not contain zinc hydroxyapatite.

In another aspect, the present invention relates to a composition comprising 0.001 to 40 wt.-% of hydroxyapatite,
0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof,
0.001 to 5 wt.-% of a surfactant, and
0.001 to 5 wt.-% of a polyalcohol selected from erythritol, arabitol, lactitol, maltitol, mannitol, sorbitol, xylitol, and mixtures thereof, wherein the composition does not contain zinc hydroxyapatite.

The present invention also relates to a kit of parts comprising the composition of the present invention and a spraying device for applying the composition to the mucous membrane.

### Detailed description

The following definitions are relevant in connection with the embodiments of the present invention.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the composition specified by "consisting of".

The term "weight-%" or "wt.-%" is to be understood to refer to the weight amount of the component relative to the total weight amount of the respective composition, if not specified otherwise.

The term "water-soluble" refers to a zinc salt that has a solubility of 0.1 g/L or more at standard conditions. Correspondingly, the term "water-insoluble" refers to a zinc-salt that has a solubility of less than 0.1 g/L at standard conditions, i.e. at a temperature of 15 °C and a pH value of 7.0.

The term "film-forming" polymer refers to a polymer that results in a cohesive, continuous layer when applied to a surface. In the present invention, when the composition is applied topically to the mucous membrane, the film-forming polymer leads to the formation of a cohesive layer of the composition that further has enhanced adhesive properties to the mucous membrane.

As used herein, the term "prevention" of a virus infection does not only refer to prevention of an infection for the subject that uses the composition of the present invention but also refers to the prevention of passing the infection on to further subjects, in case the subject that uses the composition is already infected with the virus. Thus, a dual method of preventing widespread of the virus is achieved by the composition of the present invention. Additionally, the composition has antiviral activity so that the virus count is decreased in the throat and nasal area, thereby resulting in an effective local treatment of the virus, as the virus is predominately located in these areas.

Preferred embodiments according to the invention are defined hereinafter. The preferred embodiments are preferred alone or in combination. Further, it is to be understood that the following preferred embodiments refer to all aspects of the present invention, i.e. the compound for use, the compound, and the kit of parts.

In an embodiment, the invention relates to a composition comprising 0.001 to 40 wt.-% of hydroxyapatite for use in the treatment and/or prevention of a virus infection. In a preferred embodiment, the composition comprises 0.01 to 20 wt.%, 0.05 to 10, or 0.1 to 5 wt.-% hydroxyapatite.

The above composition is useful for both, the treatment as well as the prevention of a virus infection. In a preferred embodiment, the composition is used for the prevention of a virus infection.

The hydroxyapatite is a naturally occurring inorganic mineral having the general formula Ca₅(PO₄)₃(OH). In a preferred embodiment, the hydroxyapatite is not doped with any ions, i.e. the hydroxyapatite has less than 1 wt-% of its ions (Ca²⁺, PO₄³⁻ und OH-) replaced by different ions.

Hydroxyapatite is naturally occurring but can also be obtained by processes known to the skilled person. For example, hydroxyapatite can be obtained by the reaction of a calcium salt, such as CaCl₂ or CaNO₃, with a phosphate salt, such as (NH₄)₂PO₄, Na₂PO₄ or NaHPO₄, in water at a temperature of 10 to 100 °C or in an autoclave at temperatures above 100 °C.

Hydroxyapatite can i.a. be doped with the following ions: CO₃²⁻, Ag⁺, Mg²⁺, Cu²⁺. Therefore, in a non-limiting example, the term "hydroxyapatite" is to be understood to encompass compositions doped with the above ions and to have the following formula:
Ca₁₀₋ₓMₓ(PO₄)_{6-y}(CO₃)_{y+z}(OH)_{2-z},
wherein M Cu, Mg or Ag,
x is a number between 0 and 0.6,
y is a number between 0 and 0.9, and
z is a number between 0 and 0.32.

In a preferred embodiment, x is a number between 0.0055 and 0.6, y is a number between 0.065 and 0.9, and z is a number between 0 and 0.32.

This doping with ions can be obtained by the addition of suitable salts, such as MgCl₂, during the formation of the hydroxyapatite.

The composition of the present invention does not contain zinc hydroxyapatite, i.e. the hydroxyapatite of the present invention does not contain zinc ions.

In a non-limiting example, the hydroxyapatite has a volume-based particle size X₅₀ of 1 to 1000 nm, 10 to 500 nm, 20 to 300 nm 30 to 200 nm or 50 to 150 nm, preferably 30 to 200 nm. The X₅₀ value can be measured by laser diffraction using a Mastersizer 2000 (Malvern Instruments GmbH) according to DIN ISO 14887:2010.

In a non-limiting example, the hydroxyapatite has a crystalline form having a hexagonal crystal lattice with an *a*-axis of 0.930 to 0.950 nm, preferably of 0.933 to 0.948 nm, particularly preferably of 0.936 to 0.945 nm and a length of the c-axis of 0.680 bis 0.700 nm, preferably of 0.682 to 0.696 nm, particularly preferably of 0.685 to 0.692 nm. The lattice parameters can be determined by X-ray powder diffraction using a diffractometer (Bruker D8) and Rietveld analysis.

In an embodiment, the composition further comprises 0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof.

The additional presence of zinc ions has been found to further increase the antiviral activity of the present invention.

The water-soluble zinc salt also encompasses solvates (such as hydrates) of the respective zinc salts. It is preferred that the composition comprises water. Therefore, it is not particularly relevant which specific solvate is used.

In a preferred embodiment, the composition comprises the water-soluble zinc salt in an amount of 0.01 to 10 wt.-%, 0.05 to 5 wt.-% or 0.05 to 1 wt.-%, preferably in an amount of 0.05 to 0.5 wt.-%.

The composition of the present invention further comprises 0.001 to 5 wt.-% of a surfactant, preferably 0.001 to 1 wt.-% of a surfactant. The surfactant can be an ionic or non-ionic surfactant. It has surprisingly been found that the composition of the present invention is effective against viruses, even when small amounts of 0.001 to 1 wt.-% of a surfactant are used. It is believed that the surfactant acts synergistically with the hydroxyapatite by stabilizing the hydroxyapatite and forming stable interface structures that deactivate the virus envelope more efficiently.

In a preferred embodiment, the surfactant is selected from alkyl sulfates, alkyl sarcosinates, alkyl taurates, and mixtures thereof. It is to be understood by the person skilled in the art that the above terms refer to charge neutral compounds containing a counter cation M, such as sodium.

Alkyl sulfates have the formula ROSO₃M , alkyl sarcosinates have the formula RC(O)N(CH₃)CH₂CO₂M, and taurates have the formula RC(O)N(CH₃)CH₂CH₂SO₃M, wherein R is a C₄-C₂₆ alkyl or C₄-C₂₆ alkenyl, and M is a water-soluble cation such as ammonium, sodium, potassium or triethanolamine. Preferably, R is C₁₂-C₁₆ alkyl or C₁₂-C₁₈.

In an embodiment, the composition comprises a non-ionic surfactant selected from polyoxyethylene fatty acid ethers, polyglycerol ethers of fatty acids, polyglycerol esters of fatty acids, and mixtures thereof.

In a particularly preferred embodiment, the composition comprises 0.001 to 1 wt.-% of a surfactant selected from alkyl sulfates, alkyl sarcosinates, alkyl taurates, and mixtures thereof.

In a preferred embodiment, the composition comprises 0.001 to 1 wt.-% of sodium lauryl sulfate. In a preferred embodiment, the composition comprises 0.001 to 1 wt.-% sodium myristoyl sarcosinate. In a preferred embodiment, the composition comprises 0.001 to 1 wt.-% sodium methyl cocoyl taurate. In a preferred embodiment, the composition comprises 0.001 to 1 wt.-% of sodium lauryl sulfate, 0.001 to 1 wt.-% sodium myristoyl sarcosinate, and 0.001 to 1 wt.-% sodium methyl cocoyl taurate. In an even more preferred embodiment, the composition comprises 0.001 to 1 wt.-% of a surfactant selected from sodium lauryl sulfate, sodium myristoyl sarcosinate, sodium methyl cocoyl taurate, and mixtures thereof.

In an embodiment, the composition comprises 0.001 to 5 wt.-% of a polyalcohol selected from erythritol, arabitol, lactitol, maltitol, mannitol, sorbitol, xylitol, and mixtures thereof. Polyalcohols can act as flavoring agents, in particular as sweeteners or masking agents that mask an otherwise unpleasant taste while at the same time minimizing or preventing tooth decay. Thus, the use of polyalcohols, such as xylitol, has the bonus effect that tooth decay due to dental caries is minimized and/or prevented, as cariogenic bacteria of the family *Streptococcus mutans* cannot metabolize polyalcohols. It is preferred that the composition comprises sorbitol and/or xylitol.

The composition may comprise 0.001 to 60 wt.-% ethanol. In an alternative embodiment, the composition of the present composition does not comprise ethanol. Ethanol has disinfectant and antiviral properties. The mouth washes and compositions for spraying of the prior art that are believed to be effective as antiviral compositions all contain ethanol. It is evident from Steinmann et al., The Journal of Infectious Diseases 2020, XX, pp 1-4 that only those compositions containing ethanol show *in vitro* activity against SARS-CoV-2. In view thereof, it was unexpected that the present composition is effective in the treatment and prevention of a virus infection, in particular in the treatment and prevention of a Corona virus infection, even in the absence of ethanol. Thus, it was surprising that the present composition that does not comprise ethanol is still an effective antiviral composition.

The composition may additionally contain a flavoring agent. Non-limiting examples of flavoring agents are sweeteners and essential oils. Sweeteners can be polyalcohols, aspartame, saccharin, and corn syrup. Non-limiting examples of essential oils include menthol, peppermint oil, and spearmint oil. In an embodiment, the composition comprises 0.001 to 5 wt.-% of an essential oil selected from eucalyptol, thymol, menthol, anise oil, fennel oil, and levomenthol. These essential oils can have additional beneficial properties and act as antiviral and antipuritic agents.

In an embodiment, the composition comprises a transferrin, preferably lactoferrin. These proteins can further enhance the antiviral properties of the present composition. Without being bound to theory, it is believed that transferrins and hydroxyapatite's antiviral effects are based on different modes of action. Therefore, the combination of transferrin and hydroxyapatite results in a synergistic effect.

In an embodiment, the composition comprises a film-forming polymer selected from carrageenan, carboxymethyl cellulose (CMC) and pharmaceutically acceptable salts thereof, hydroxypropyl methylcellulose (HPMC), ethyl cellulose, methyl cellulose, hydroxyethyl cellulose, hydroxypropyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, and mixtures thereof.

Non-limiting examples of the above pharmaceutically acceptable salts encompass the salts with calcium, sodium, potassium, and mixtures thereof.

Pharmaceutically acceptable salts of hyaluronic acid comprise the sodium and potassium salt.

It is believed that the present invention functions by forming a protective layer of the composition according to the present invention on the mucous membrane in the mouth and/or nose. This antiviral drug protective layer effectively prevents infection with a virus by droplet infection in subjects not yet infected. In addition, the composition also reduces the virus count in the respiratory tract and in addition, a protective layer is formed so that a subject already infected is less infectious to other subjects, thereby also preventing further infections.

In addition, the film-forming polymer stabilizes the hydroxyapatite composition during storage and further increases the time of contact between the hydroxyapatite and the mucous membrane in the mouth and/or nose by forming a stable and long-lasting active layer of hydroxyapatite on the mucous membrane.

The copolymer of polyvinyl ether and maleic acid is preferable an alternating copolymer. It is to be understood that the polymer is derived from monomer units of methyl vinyl ether and maleic acid or maleic anhydride and the copolymer is also called poly(methyl vinyl ether-co-maleic acid). This polymer can be obtained by e.g. radical polymerization of the monomer units and copolymers of methyl vinyl ether and maleic acid are commercially available as Gantrez^{™} S polymer (International Nomenclature of Cosmetic Ingredients: PVM/MA copolymer; Ashland Inc.).

Pharmaceutically acceptable salts of this copolymer can be alkali metals and/or earth alkali metals. Thus, the salts can be selected from lithium, sodium, potassium, magnesium, calcium, and mixtures thereof. It is preferred that the salt is a sodium/calcium salt. Calcium sodium copolymer of poly(methyl vinyl ether-co-maleic acid) are commercially available as Gantrez^{™} MS-955 polymer (International Nomenclature of Cosmetic Ingredients: calcium/sodium PVM/MA copolymer Ashland Inc.).

In an embodiment, the composition comprises the film forming polymer in an amount of 0.01 to 10 wt.-%, 0.1 to 5 wt.-% or 1 to 2 wt.-%, preferably in an amount of 0.5 to 3 wt.-%.

In an embodiment, the composition further comprises a water-insoluble zinc salt. In an embodiment, the water-insoluble salt is present in an amount of 0.001 to 20 wt.-%. In a preferred embodiment, the composition comprises the water-insoluble zinc salt in an amount of 0.01 to 10 wt.-%, 0.05 to 5 wt.-% or 0.05 to 1 wt.-%, preferably in an amount of 0.05 to 0.5 wt.-%.

In yet another embodiment, the water-insoluble zinc salt is zinc oxide.

In an embodiment, the composition further comprises an additional active ingredient selected from menthol, lactoferrin, sorbitol, xylitol, saccharin, and mixtures thereof. These ingredients can have anti-inflammatory properties and/or protective properties of the skin and further enhance treatment and/or prevention of a virus infection. These additional active ingredients are preferably present in an amount of 0.001 to 10 wt.-%, 0.01 to 5 or 0.05 to 2 wt.-%.

In another embodiment, the composition may comprise preservatives, such as benzoic acid, phenoxyethanol, citric acid, lactic acid, ascorbic acid, tocopherol, as well as pharmaceutically acceptable salts thereof.

The composition is administered as a mouth rinse or as an aerosol by spraying. In an embodiment, the composition is applied topically by spraying the composition on the respective area. This application has the advantage that a uniform, homogenous and long-lasting protective layer on the mucous membrane is formed. In addition, protection can be achieved on a large area, thereby leading to an even better protection.

In an embodiment, the composition is applied to the mucous membrane, preferable using a spraying device.

The spraying device can be used to apply the composition to the skin of e.g. the hands or to the mucous membrane in the mouth, pharynx or nose. It is preferred that the spraying device is used to apply the composition to the mucous membrane in the mouth or pharynx. It is particularly preferred that the spraying device contains a means for (pre-)determining the amount of the composition that is applied.

Suitable pump spraying devices are known (see i.a. US 4,245,967) and are commercially available as "throat sprays". Such sprays are usually used to treat sore throats caused by bacterial infections of the throat and mucous membrane and/or decrease any symptoms of such infections. Such sprays can contain an antibacterial ingredient to fight the bacterial infection as well as an analgesic to decrease any symptoms of pain and/or soreness.

Spraying devices commonly comprise a feed chamber and an outlet through which the composition is sprayed in a predetermined amount by a suitable means for spraying the composition through a nozzle. It is preferred that the spraying device is a pump spraying device. Such a pump spray contains an atomizer nozzle that disperses the liquid composition as a fine aerosol. This aerosol covers the area on which it is sprayed and forms a thin film.

In an embodiment, the virus belongs to the family of *Coronaviridae,* and preferably is SARS-CoV-2. In another embodiment, the virus belongs to the family of *Orthomyxoviridae, Paramyxoviridae, Filoviridae,* or *Picornaviridae.*

### Examples

### Example 1:

The composition comprising the components as depicted below in Table 1 was obtained by mixing the components. N.B. the amount of polyalcohol exceeds the amount defined in claim 14.

**Table 1: Ingredients of Example 1**

| **Ingredients** | **Amount in wt.-** |
|---|---|
| | **%** |
| Aqua | add. 100 |
| Sorbitol | 15 |
| Xylitol | 1.5 |
| Hydroxyapatite | 0.62 |
| Phenoxyethanol | 0.3 |
| Sodium Benzoate | 0.15 |
| Zinc PCA | 0.3 |
| PEG-40 hydrogenated castor oil | 0.7 |
| Cellulose gum | 0.6 |
| Sodium lauryl sulfate | 0.7 |
| Sodium myristoyl sarcosinate | 0.05 |
| Sodium methyl cocoyl taurate | 0.09 |
| Sodium saccharin | 0.05 |
| Lactoferrin | 0.03 |
| Sodium hyaluronate | 0.1 |
| Phosphoric acid | 0.1 |

### Example 2:

The composition of Example 1 was measured for antiviral efficacy against bovine Corona virus (BCoV) according to DIN EN 14476.

After treatment with a composition of Example 1 and a contact time of 30 sec, the virus load of bovine Corona virus was reduced to an undetectable level. Thus, the virus was completely inactivated by the composition of Example 1.

## Claims

1. A composition comprising 0.001 to 40 wt.-% of hydroxyapatite for use in the treatment and/or prevention of an enveloped virus infection, further comprising 0.001 to 5 wt.-% of a surfactant, wherein the composition is administered as a mouth rinse or as an aerosol by spraying, wherein the composition does not contain zinc hydroxyapatite.

2. The composition for use according to claim 1, further comprising 0.001 to 5 wt.-% of a polyalcohol selected from erythritol, arabitol, lactitol, maltitol, mannitol, sorbitol, xylitol, and mixtures thereof.

3. The composition for use according to claims 1 or 2, comprising 0.001 to 1 wt.-% of the surfactant.

4. The composition for use according to any one of claims 1 to 3, comprising 0.001 to 1 wt.-% of a surfactant selected from sodium lauryl sulfate, sodium myristoyl sarcosinate, sodium methyl cocoyl taurate, and mixtures thereof.

5. The composition for use according to claim 2, wherein the polyalcohol is sorbitol and/or xylitol.

6. The composition for use according to any one of claims 1 to 5, wherein the composition does not comprise ethanol or does comprise 0.001 to 60 wt.-% ethanol.

7. The composition for use according to any one of claims 1 to 6, further comprising 0.001 to 5 wt.-% of eucalyptol, thymol, menthol, anise oil, fennel oil, or levomenthol.

8. The composition for use according to any one of claims 1 to 7, further comprising 0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof.

9. The composition for use according to any one of claims 1 to 8, wherein the composition comprises a transferrin, preferably lactoferrin.

10. The composition for use according to any one of claims 1 to 9, further comprising a film-forming polymer selected from carrageenan, carboxymethyl cellulose (CMC) and pharmaceutically acceptable salts thereof, hydroxypropyl methylcellulose (HPMC), ethyl cellulose, methyl cellulose, hydroxyethyl cellulose, hydroxypropyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, and mixtures thereof.

11. The composition for use according to any one of claims 1 to 10, further comprising a water-insoluble zinc salt.

12. The composition for use according to any one of claims 1 to 11, wherein the composition is applied to the mucous membrane, preferable using a spraying device.

13. The composition for use according to any one of claims 1 to 12, wherein the virus belongs to the family of *Coronaviridae,* and preferably is SARS-CoV-2.

14. A composition having antiviral activity against enveloped viruses comprising
0.001 to 40 wt.-% of hydroxyapatite,
0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof,
0.001 to 5 wt.-% of a surfactant, and
0.001 to 5 wt.-% of a polyalcohol selected from erythritol, arabitol, lactitol, maltitol, mannitol, sorbitol, xylitol, and mixtures thereof,
wherein the composition does not contain zinc hydroxyapatite.

15. A kit of parts comprising the composition for use according to any one of claims 1 to 13 and a spraying device for applying the composition to the mucous membrane.

## Patentansprüche

1. Zusammensetzung, umfassend 0,001 bis 40 Gew.-% Hydroxylapatit zur Verwendung bei der Behandlung und/oder Vorbeugung einer Infektion mit einem behüllten Virus, ferner umfassend 0,001 bis 5 Gew.-% eines Tensids, wobei die Zusammensetzung als Mundspülung oder als Aerosol durch Sprühen verabreicht wird, wobei die Zusammensetzung kein Zinkhydroxylapatit enthält.

2. Zusammensetzung zur Verwendung nach Anspruch 1, ferner umfassend 0,001 bis 5 Gew.-% eines Polyalkohols, ausgewählt aus Erythrit, Arabit, Lactit, Maltit, Mannit, Sorbit, Xylit und Mischungen davon.

3. Zusammensetzung zur Verwendung nach Anspruch 1 oder 2, umfassend 0,001 bis 1 Gew.-% des Tensids.

4. Zusammensetzung zur Verwendung nach einem der Ansprüche 1 bis 3, umfassend 0,001 bis 1 Gew.-% eines Tensids, ausgewählt aus Natriumlaurylsulfat, Natriummyristoylsarkosinat, Natriummethylcocoyltaurat und Mischungen davon.

5. Zusammensetzung zur Verwendung nach Anspruch 2, wobei der Polyalkohol Sorbit und/oder Xylit ist.

6. Zusammensetzung zur Verwendung nach einem der Ansprüche 1 bis 5, wobei die Zusammensetzung kein Ethanol oder 0,001 bis 60 Gew.-% Ethanol enthält.

7. Zusammensetzung zur Verwendung nach einem der Ansprüche 1 bis 6, ferner umfassend 0,001 bis 5 Gew.-% Eukalyptol, Thymol, Menthol, Anisöl, Fenchelöl oder Levomenthol.

8. Zusammensetzung zur Verwendung nach einem der Ansprüche 1 bis 7, ferner umfassend 0,001 bis 20 Gew.-% eines wasserlöslichen Zinksalzes, ausgewählt aus der Gruppe bestehend aus Zink-L-pyrrolidoncarboxylat, Zinkacetat, Zinkchlorid, Zinkhistidin, Zinkgluconat, Zinkaspartat, Zinkcitrat, Zinksulfat, Zinklactat und Mischungen davon.

9. Zusammensetzung zur Verwendung nach einem der Ansprüche 1 bis 8, wobei die Zusammensetzung ein Transferrin, vorzugsweise Lactoferrin, enthält.

10. Zusammensetzung zur Verwendung nach einem der Ansprüche 1 bis 9, ferner umfassend ein filmbildendes Polymer, ausgewählt aus Carrageen, Carboxymethylcellulose (CMC) und pharmazeutisch verträglichen Salzen davon, Hydroxypropylmethylcellulose (HPMC), Ethylcellulose, Methylcellulose, Hydroxyethylcellulose, Hydroxypropylcellulose, Hyaluronsäure und pharmazeutisch verträglichen Salzen davon, Copolymeren von Methylvinylether und Maleinsäure und pharmazeutisch verträglichen Salzen davon, Carboxymethylchitin, Polyvinylpyrrolidon, Chitosan und Mischungen davon.

11. Zusammensetzung zur Verwendung nach einem der Ansprüche 1 bis 10, ferner umfassend ein wasserunlösliches Zinksalz.

12. Zusammensetzung zur Verwendung nach einem der Ansprüche 1 bis 11, wobei die Zusammensetzung auf die Schleimhaut aufgetragen wird, vorzugsweise unter Verwendung einer Sprühvorrichtung.

13. Zusammensetzung zur Verwendung nach einem der Ansprüche 1 bis 12, wobei das Virus zur Familie der *Coronaviridae* gehört und vorzugsweise SARS-CoV-2 ist.

14. Zusammensetzung mit antiviraler Aktivität gegen behüllte Viren, umfassend
0,001 bis 40 Gew.-% Hydroxylapatit,
0,001 bis 20 Gew.-% eines wasserlöslichen Zinksalzes, ausgewählt aus der Gruppe bestehend aus Zink-L-pyrrolidoncarboxylat, Zinkacetat, Zinkchlorid, Zinkhistidin, Zinkgluconat, Zinkaspartat, Zinkcitrat, Zinksulfat, Zinklactat und Mischungen davon,
0,001 bis 5 Gew.-% eines Tensids und
0,001 bis 5 Gew.-% eines Polyalkohols, ausgewählt aus Erythrit, Arabit, Lactit, Maltit, Mannit, Sorbit, Xylit und Mischungen davon,
wobei die Zusammensetzung kein Zinkhydroxylapatit enthält.

15. Baukastensystem, umfassend die Zusammensetzung zur Verwendung nach einem der Ansprüche 1 bis 13 und eine Sprühvorrichtung zum Auftragen der Zusammensetzung auf die Schleimhaut.

## Revendications

1. Composition comportant 0,001 à 40 % en poids d'hydroxyapatite pour une utilisation dans le traitement et/ou la prévention d'une infection par virus enveloppé, comportant en outre 0,001 à 5 % en poids d'un tensioactif, dans laquelle la composition est administrée en bain de bouche ou en aérosol par pulvérisation, dans laquelle la composition ne contient pas d'hydroxyapatite de zinc.

2. Composition pour une utilisation selon la revendication 1, comportant en outre 0,001 à 5 % en poids d'un polyalcool choisi parmi l'érythritol, l'arabitol, le lactitol, le maltitol, le mannitol, le sorbitol, le xylitol et des mélanges de ceux-ci.

3. Composition pour une utilisation selon les revendications 1 ou 2, comportant 0,001 à 1 % en poids du tensioactif.

4. Composition pour une utilisation selon l'une quelconque des revendications 1 à 3, comportant 0,001 à 1 % en poids d'un tensioactif choisi parmi le lauryl sulfate de sodium, le myristoyl sarcosinate de sodium, le méthyl cocoyl taurate de sodium et des mélanges de ceux-ci.

5. Composition pour une utilisation selon la revendication 2, dans laquelle le polyalcool est le sorbitol et/ou le xylitol.

6. Composition pour une utilisation selon l'une quelconque des revendications 1 à 5, dans laquelle la composition ne comporte pas d'éthanol ou comporte 0,001 à 60 % en poids d'éthanol.

7. Composition pour une utilisation selon l'une quelconque des revendications 1 à 6, comportant en outre 0,001 à 5 % en poids d'eucalyptol, de thymol, de menthol, d'essence d'anis, d'essence de fenouil ou de lévomenthol.

8. Composition pour une utilisation selon l'une quelconque des revendications 1 à 7, comportant en outre 0,001 à 20 % en poids d'un sel de zinc soluble dans l'eau choisi parmi le groupe constitué de zinc-L-pyrrolidone-carboxylate, d'acétate de zinc, de chlorure de zinc, d'histidine de zinc, de gluconate de zinc, d'aspartate de zinc, de citrate de zinc, de sulfate de zinc, de lactate de zinc et de mélanges de ceux-ci.

9. Composition pour une utilisation selon l'une quelconque des revendications 1 à 8, dans laquelle la composition comporte une transferrine, de préférence une lactoferrine.

10. Composition pour une utilisation selon l'une quelconque des revendications 1 à 9, comportant en outre un polymère filmogène choisi parmi le carraghénane, la carboxyméthyl cellulose (CMC) et des sels de celle-ci acceptables du point de vue pharmaceutique, l'hydroxypropyl méthylcellulose (HPMC), l'éthyl cellulose, la méthyl cellulose, l'hydroxyéthyl cellulose, l'hydroxypropyl cellulose, l'acide hyaluronique et des sels de celui-ci acceptables du point de vue pharmaceutique, des copolymères d'éther méthylvinylique et d'acide maléique, et des sels de ceux-ci acceptables du point de vue pharmaceutique, la carboxyméthyl chitine, la polyvinylpyrrolidone, le chitosan et des mélanges de ceux-ci.

11. Composition pour une utilisation selon l'une quelconque des revendications 1 à 10, comportant en outre un sel de zinc insoluble dans l'eau.

12. Composition pour une utilisation selon l'une quelconque des revendications 1 à 11, dans laquelle la composition est appliquée sur la membrane muqueuse, en utilisant de préférence un dispositif de pulvérisation.

13. Composition pour une utilisation selon l'une quelconque des revendications 1 à 12, dans laquelle le virus appartient à la famille de *Coronaviridae,* et est de préférence le SARS-CoV-2.

14. Composition ayant une activité antivirale à l'encontre des virus enveloppés, comportant :
0,001 à 40 % en poids d'hydroxyapatite,
0,001 à 20 % en poids d'un sel de zinc soluble dans l'eau choisi parmi le groupe constitué de zinc-L-pyrrolidone-carboxylate, d'acétate de zinc, de chlorure de zinc, d'histidine de zinc, de gluconate de zinc, d'aspartate de zinc, de citrate de zinc, de sulfate de zinc, de lactate de zinc et de mélanges de ceux-ci,
0,001 à 5 % en poids d'un tensioactif, et
0,001 à 5 % en poids d'un polyalcool choisi parmi l'érythritol, l'arabitol, le lactitol, le maltitol, le mannitol, le sorbitol, le xylitol et des mélanges de ceux-ci,
dans laquelle la composition ne contient pas d'hydroxyapatite de zinc.

15. Kit de parties comportant la composition pour une utilisation selon l'une quelconque des revendications 1 à 13, et un dispositif de pulvérisation pour appliquer la composition sur la membrane muqueuse.
